# EUROPEAN PATENT APPLICATION

(11) **EP 4 417 145 A1**
(43) Date of publication of application: **21.08.2024**
(21) Application number: 22880321.9
(22) Date of filing: 11.10.2022
(51) Int. Cl.: A61B 17/32

(54) **ULTRASONIC SCALPEL**

(30) Priority: 12.10.2021 CN 202111185005
(71) Applicant: B. J. Zh. F. Panther Medical Equipment Co., Ltd., Beijing 102200 (CN)
(72) Inventor: LIU, Qing, Beijing 102200 (CN); YAO, Yinfeng, Beijing 102200 (CN); ZHOU, Qingjie, Beijing 102200 (CN); GU, Linfei, Beijing 102200 (CN)
(74) Representative: Huang, Liwei
(86) International application number: PCT/CN2022/124724
(87) International publication number: WO 2023/061384

(57) **Abstract**

The embodiment of the invention discloses an ultrasonic knife which comprises a driving handle, a knife head assembly and a hollow motor; the knife head assembly comprises a knife rod and a knife head arranged at one end of the knife rod, a cavity is provided at the other end of the knife rod, an external thread is arranged at one end, close to the knife rod, of the driving handle, and an internal thread adapted with the external thread is arranged at the cavity; the hollow motor is provided with a passage through which the knife rod can pass, and the hollow motor can drive the knife rod to rotate under the condition that the knife rod can pass through the hollow motor. The one end that the knife rod provided with the cavity passes hollow motor, makes the external thread of driving handle contact with the internal thread in the cavity, then starts hollow motor drive the knife rod to rotate, makes knife rod connect by thread to driving handle, an operator does not need to manually rotate the knife rod, and the time and labor are saved.

## Description

### TECHNICAL FIELD

The invention relates to the field of medical instruments, in particular to an ultrasonic knife.

### BACKGROUND

Ultrasonic knife systems have become commonly used in clinical surgical procedures. The ultrasonic knife mainly comprises a host, a driving handle, a connecting wire, an ultrasonic knife, a pedal and the like; the working principle is that the pedal or hand-held ultrasonic knife is used for activation, at the moment, the host outputs electric energy under the resonance frequency of the vibration system to the driving handle, the driving handle converts the electric energy into mechanical energy and outputs the mechanical energy to the knife head, and the knife head further amplifies the vibration to carry out mechanical vibration, so that water in tissue cells is vaporized, protein hydrogen bonds are broken, the cells are disintegrated, the tissues are cut or coagulated, and the purposes of cutting the tissues and stopping bleeding are achieved.

Ultrasonic knife includes a driving handle and a knife head assembly, and the driving handle is generally connected detachably with the knife head assembly in order to take and store, but the existing knife body and knife head assembly require that the operator manually assembles, and the time and labor are saved.

### SUMMARY

The embodiment of the invention provides an ultrasonic knife, which aims to solve the problem that the existing knife body and knife head assembly need to be manually assembled by an operator, and the time and labor are saved.

The embodiment of the invention provides an ultrasonic knife, which comprises a driving handle, a knife head assembly and a hollow motor;

the knife head assembly comprises a knife rod and a knife head arranged at one end of the knife rod, a cavity is provided at the other end of the knife rod, an external thread is arranged at one end, close to the knife rod, of the driving handle, and an internal thread adapted with the external thread is arranged within the cavity;

the hollow motor is internally provided with a passage through which the knife rod can pass, and the hollow motor can drive the knife rod to rotate under the condition that the knife rod can pass through the hollow motor.

Specially, a camera assembly is further disposed on a portion of the knife rod, which is close to the knife head, the camera assembly comprises a camera portion, a driving portion and a transmission portion, the camera portion is connected with the driving portion through the transmission portion, and the driving portion drives the camera portion to rotate around the knife rod through the transmission portion.

Specially, the transmission portion comprises a rotating sleeve arranged outside the knife rod, the rotating sleeve is internally provided with a moving gear and a stationary gear, the stationary gear is sleeved outside the knife rod, the driving portion is arranged on the rotating sleeve, and the driving portion is connected with the moving gear; the camera portion is arranged on the outer wall of the rotating sleeve.

Specially, the camera portion is detachably connected to the rotating sleeve.

Specially, the camera portion comprises a first bolt, a camera and a hoop sleeved outside the camera, the hoop is provided with a first screw hole adapted with the bolt, the rotating sleeves are provided with second screw holes adapted with the bolt, and the first screw hole is connected with the second screw hole through the first bolt.

Specially, the number of the second screw holes is at least two, and at least two of the second screw holes are sequentially arranged from top to bottom along the axial direction of the rotating sleeve.

Specially, the rotating sleeve comprises a first cover, a second cover and a peripheral wall, and the peripheral wall is detachably connected with the first cover and the second cover respectively.

Specially, the ultrasonic knife further comprises a second bolt and a third bolt; a third screw hole adapted with the second bolt is provided at one end face of the peripheral wall, a fourth screw hole adapted with the third bolt is provided at the other end face of the peripheral wall, a fifth screw hole adapted with the third bolt is provided at the first cover, and a sixth screw hole adapted with the third bolt is provided at the second cover; the third screw hole is connected with the fifth screw hole through the second bolt, and the fourth screw hole is connected with the sixth screw hole through the third bolt.

Specially, a conducting end is provided on the driving handle and the knife rod;

under the condition that the driving handle is connected with the knife rod, the conducting end of the driving handle contacts with the conducting end in the knife rod, so as to transmit the electric energy of the driving handle to the hollow motor through the knife rod to supply power for the hollow motor.

Specially, the ultrasonic knife further comprises a motor power wire connected to the hollow motor.

According to an ultrasonic knife provided by the embodiment of the invention, one end of the knife rod, which is provided with the cavity, passes through the hollow motor, so that the external thread of the driving handle contacts with the internal thread in the cavity, and then the hollow motor is started to drive the knife rod to rotate, so that the knife rod is connected with the driving handle by thread, an operator does not need to manually rotate the knife rod, and the time and labor are saved.

### BRIEF DESCRIPTION OF THE DRAWINGS

The following drawings of the invention are included to provide a further understanding of the invention as a part of the embodiments. The drawings illustrate embodiments of the invention and, together with the description, serve to explain the principle of the invention.

In the drawings:
FIG. 1 is a perspective view of an ultrasonic knife in accordance with an alternative embodiment of the present invention;
FIG. 2 is a partial cross-sectional view of FIG. 1;
FIG. 3 is an exploded view of FIG. 2;
FIG. 4 is a perspective view of FIG. 2;
FIG. 5 is a partial enlarged view of FIG. 1;
FIG. 6 is a block diagram of a camera assembly;
FIG. 7 is an exploded view of FIG. 5.

wherein, 1-a knife head assembly, 101-a knife rod, 102-a knife head, 2-a camera assembly, 201-a driving portion, 202-a transmission portion, 2021-a stationary gear, 2022-a moving gear, 2023-a rotating sleeve, 20231-a first cover, 20232-a peripheral wall, 20233-a second cover, 2024-a second screw hole, 2025-a first bolt, 203-a camera portion, 2031-a camera , 2032-a hoop, 3-a shell, 4-a hollow motor, 5-a switch, 6-a driving handle power wire, 7-a motor power wire, 8-a driving handle, 9-a wire and a video transmission line, 10 a wire and a video transmission line sleeve.

### DETAILED DESCRIPTION

In the following description, numerous specific details are set forth in order to provide a more thorough understanding of the present invention. It will be apparent, however, to one skilled in the art, that the present invention may be practiced without one or more of these specific details. In other instances, well-known features have not been described in order to avoid obscuring the invention.

It should be noted that the terminology used herein is for the purpose of describing particular embodiments only and is not intended to be limiting of exemplary embodiments according to the invention. As used herein, the singular is intended to include the plural unless the context clearly dictates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or combination thereof.

Exemplary embodiments according to the present invention will now be described in more detail with reference to the accompanying drawings. These exemplary embodiments may, however, be embodied in many different forms and should not be construed as limited to only the embodiments set forth herein. It is to be understood that these embodiments are provided so that this disclosure will be thorough and complete, and will fully convey the concept of these exemplary embodiments to those skilled in the art.

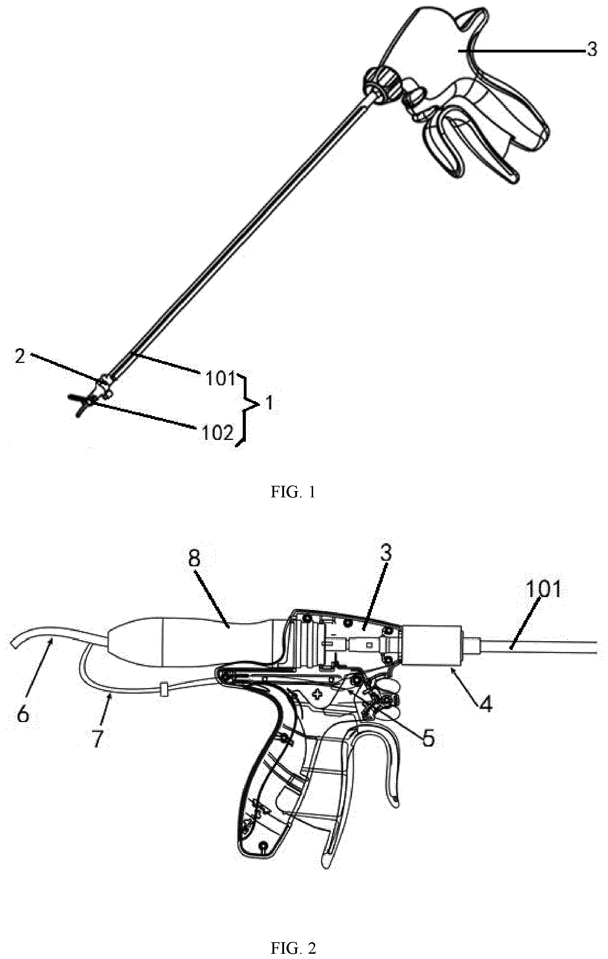

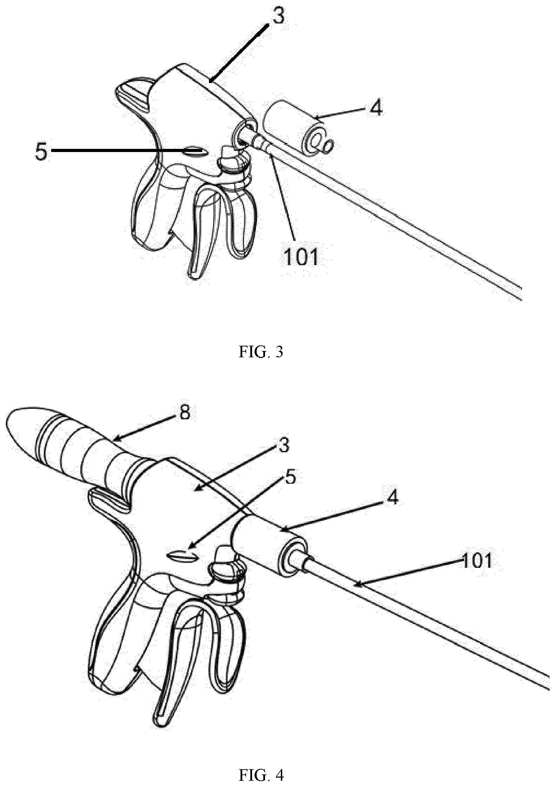

As shown in FIG. 1, 2, 3 and 4, an embodiment of the present invention provides an ultrasonic knife, which includes a driving handle 8, a knife head assembly 1 and a hollow motor 4; the knife head assembly 1 comprises a knife rod 101 and a knife head 102 arranged at one end of the a knife rod 101, a cavity is provided at the other end of the knife rod 101, an external thread is arranged at one end, close to the knife rod 101, of the driving handle 8, and an internal thread adapted with the external thread is arranged in the cavity; a passage through which the knife rod 101 can pass is provided within the hollow motor 4, and the hollow motor 4 can drive the knife rod 101 to rotate under the condition that the knife rod 101 can pass through the hollow motor 4.

In specific application, the ultrasonic knife further comprises a shell 3, one end, provided with an external thread, of the driving handle 8 extends into the shell 3, and a driving handle power wire 6 connected with a host is further arranged on the driving handle 8 to provide working voltage for the driving handle 8. The shell 3 is also provided with a switch 5 to control the opening and closing of the hollow motor 4; specifically, when the operator toggles the switch 5 to one side, the hollow motor 4 rotates in a first direction, when the operator toggles the switch 5 to the other side, the hollow motor4 rotates in a second direction (the first direction is opposite to the second direction), and when the switch 5 is located at the middle position, the hollow motor 4 stops rotating.

The overall shape of the hollow motor 4 can adopt a structure such as a cylinder, a cuboid and the like, and the embodiment is not strictly limited. The hollow motor 4 may be disposed in the shell 3, or may be directly sleeved on the knife rod 101.

When installing a knife rod 101, if the hollow motor 4 is installed on shell 3, the operator passes hollow motor 4's passageway with one end of knife rod 101 provided with the cavity earlier to make the external thread on the driving handle 8 contact with the internal thread in the cavity, then toggle switch 5 to one side to start hollow motor 4, drive the knife rod 101 through hollow motor 4 to rotate, make knife rod 101 connect with a driving rod by thread, thereby realizing the equipment of knife rod 101 and the driving rod. If the hollow motor 4 is directly sleeved on knife rod 101, the operator directly contacts the internal thread in the cavity on knife rod 101 with the external thread of driving handle, then toggles switch 5 to start hollow motor 4, drives knife rod 101 through hollow motor 4 to rotate, makes knife rod 101 connect driving rod by thread, thereby the equipment of knife rod 101 with the driving rod is realized. When the knife rod 101 is disassembled, an operator toggles the switch 5 to the other side to start the hollow motor 4, the hollow motor 4 drives the knife rod 101 to rotate, the knife rod 101 are unscrewed from the driving rod, then the knife rod 101 can be disassembled, therefore the ultrasonic knife can realize the assembly and disassembly of the knife rod 101 through the hollow motor 4 driving the rotation of the knife rod 101, and the assembly of the knife rod 101 and the driving rod by the operator is facilitated.

It will be appreciated that the operator can determine the rotation direction of the hollow motor 4, and thus determine the toggling direction of the switch 5, based on the rotation direction of the internal thread and the external thread and the operation of the loading and unloading knife rod 101.

In the prior art, when using the ultrasonic knife, before an operator clamps biological tissues such as blood vessels through the knife head 102, the angle of the knife head 102 is often required to be adjusted so as to smoothly clamp the biological tissues. The operator rotates the ultrasonic knife in a manual mode, force, a rotating angle and the like of the operator cannot be accurately controlled due to factors such as hand fatigue, and therefore the accuracy of the operation is not high. In this embodiment, before the ultrasonic knife is used, the hollow motor 4 can be started by the operator through toggling switch 5 according to the position of the biological tissues, so as to drive the knife rod 101 to rotate, so as to smoothly clamp the biological tissues, and thus the operator does not need to manually rotate the knife head 102, which not only facilitates the operation of the operator, but also ensures more accurate control of the rotation angle and the force. The direction of rotation of a particular hollow motor 4 may be determined by the direction of rotation of the knife head 102, and thus the direction in which the operator toggles the switch 5 may be determined.

The ultrasonic knife provided by this embodiment, hollow motor 4 is passed to the one end of knife rod 101 provided with the cavity, makes the external thread of driving handle 8 and the internal thread contact in the cavity, then starts hollow motor 4 and drives knife rod 101 rotate, makes knife rod 101 connect with driving handle 8 by thread, an operator does not need to manually rotate the knife rod 101, and the time and labor are saved.

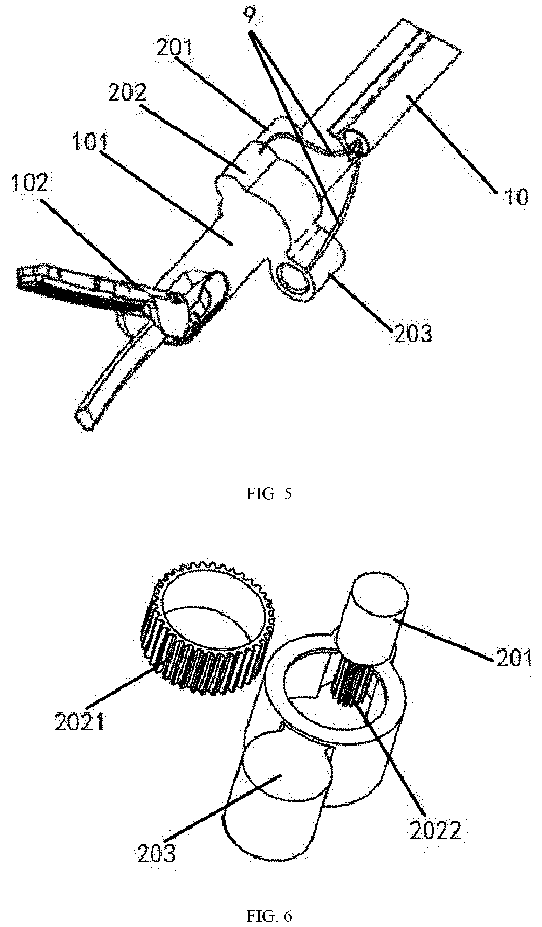

In other embodiments, as shown in FIG. 1 and 5, a camera assembly 2 is further disposed on a portion of the knife rod 101 close to the knife head 102, the camera assembly 2 includes a camera portion 203, a driving portion 201 and a transmission portion 202, the camera portion 203 is connected to the driving portion 201 through the transmission portion 202, and the driving portion 201 drives the camera portion 203 to rotate around the knife rod 101 through the transmission portion 202.

The driving portion 201 may be a motor, and existing other driving components may be used, and the present embodiment is not limited strictly. A wire and a video transmission line sleeve 10 is further disposed on one side of the knife rod 101 to accommodate a wire and a video transmission line 9 connected to the driving portion 201 and the camera portion 203.

In this embodiment, the camera assembly 2 is disposed on a portion of the knife rod 101 close to the knife head 102, and the driving portion 201 drives the camera portion 203 to rotate around the knife rod 101 through the transmission portion 202, that is, the position of the camera portion 203 can be adjusted through the transmission action between the driving portion 201 and the transmission portion 202, so that an operator can rotate the camera portion 203 to a position to be observed through the driving portion 201 and the transmission portion 202 according to actual needs to see the images of the position to be observed by the camera portion 203, thereby not only improving the shooting range of the camera portion 203, but also adjusting the position of the endoscope without the cooperation of other people, and labor are saved.

It should be noted that the camera portion 203 also rotates along with the rotation of the knife rod 101, that is, when the position of the camera portion 203 needs to be adjusted in a small range, the operator can drive the knife rod 101 to rotate through the hollow motor 4, so as to drive the camera portion 203 to rotate to reach the target position.

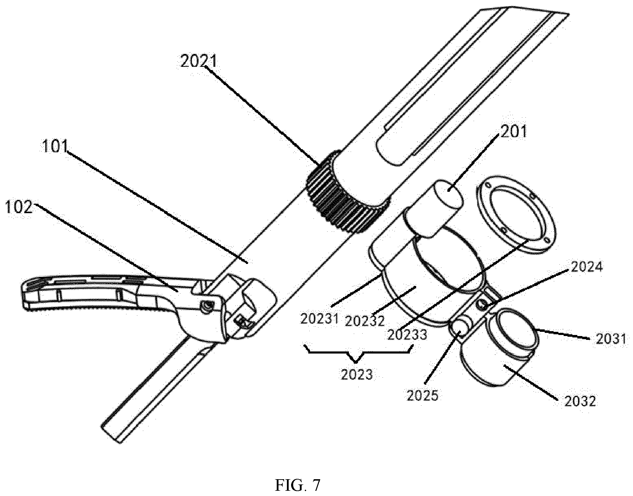

Specifically, as shown in FIG. 6 and 7, the transmission portion 202 includes a rotating sleeve 2023 disposed outside the knife rod 101, a moving gear 2022 and a stationary gear 2021 are disposed in the rotating sleeve 2023, the stationary gear 2021 is sleeved outside the knife rod 101, the driving portion 201 is mounted on the rotating sleeve 2023, and the driving portion 201 is connected to the moving gear 2022; the camera portion 203 is arranged on the peripheral wall of the rotating sleeve 2023.

When the position of the camera portion 203 is adjusted, the driving portion 201 is started, the driving portion 201 rotates to drive the moving gear 2022 to rotate, so that the moving gear 2022 rotates around the peripheral wall of the stationary gear 2021, and further the rotating sleeve 2023 rotates relative to the stationary gear 2021, that is, the rotating sleeve 2023 rotates relative to the knife rod 101, so that the camera portion 203 arranged on the rotating sleeve 2023 can be driven to rotate around the knife rod 101, and the position of the camera portion 203 can be adjusted.

Furthermore, in order to avoid the mutual winding of the wires connected to the driving portion 201 and the camera portion 203 and a video transmission line 9, the angle of rotation of the camera portion 203 around the knife rod 101 is 360 °, that is, the camera portion 203 can only rotate around the knife rod 101 by one rotation, and after the camera portion 203 rotates around the knife rod 101 by one rotation, the camera portion 203 can only rotate in the opposite direction, but cannot continue to rotate in the original direction.

Furthermore, as shown in FIG. 7, the camera portion 203 is detachably connected to the rotating sleeve 2023, thereby facilitating the operation of maintenance, sterilization, or replacement of the camera portion 203.

Specifically, the camera portion 203 includes a first bolt 2025, a camera 2031 and a hoop 2032 sleeved outside the camera 2031, the hoop 2032 is provided with a first screw hole adapted to the bolt, the rotating sleeve 2023 is provided with a second screw hole 2024 adapted to the bolt, and the first screw hole is connected to the second screw hole 2024 through the first bolt 2025.

When the camera portion 203 is mounted, the first bolt 2025 is inserted through the first screw hole and the second screw hole 2024, and then the first bolt 2025 is tightened, so that the hoop 2032 can clamp the camera 2031, and the hoop 2032 is also connected and fastened with the rotating sleeve 2023, thereby realizing the assembly of the camera portion 203 and the rotating sleeve 2023. When the camera portion 203 is detached, the hoop 2032 is separated from the rotating sleeve 2023 by unscrewing the first bolt 2025, and the camera 2031 is detached.

Furthermore, as shown in FIG. 7, the number of the second screw hole 2024 is at least two, and at least two second screw holes 2024 are sequentially arranged from top to bottom along the axial direction of the rotating sleeve 2023, so that an operator can connect the first screw hole with the second screw hole 2024 with different heights by the first bolt 2025, thereby adjusting the height of the hoop 2032 and further adjusting the height of the camera portion 203 clamped by the hoop 2032.

In the present embodiment, the height of the camera portion 203 is adjusted, so that different observation requirements in the operation can be satisfied, and the adaptability of the ultrasonic knife is provided.

Furthermore, as shown in FIG. 7, the rotating sleeve 2023 includes a first cover 20231, a second cover 20233 and a peripheral wall 20232, and the peripheral wall 20232 is detachably connected to the first cover 20231 and the second cover 20233, respectively.

By detaching the first cover 20231 and the second cover 20233, the moving gear 2022 inside the rotating sleeve 2023 and the driving portion 201 on the rotating sleeve 2023 can be detached, thereby facilitating maintenance, disinfection or replacement of the stationary gear 2021 and the driving portion 201.

Furthermore, the ultrasonic knife further comprises a second bolt and a third bolt; a third screw hole adapted with the second bolt is provided in one end face of the peripheral wall 20232, a fourth screw hole adapted with the third bolt is provided in the other end face of the peripheral wall 20232, a fifth screw hole adapted with the third bolt is provided in the first cover 20231, and a sixth screw hole adapted with the third bolt is provided in the second cover 20233; the third screw hole is connected with the fifth screw hole through a second bolt, and the fourth screw hole is connected with the sixth screw hole through a third bolt.

It is understood that the third screw hole and the fourth screw hole may be communicated, namely, run through the peripheral wall 20232; or may be disconnected, namely, the third screw hole and the fourth screw hole have certain depth, and the second bolt and the third bolt can be extended into them.

When the stationary gear 2021 and the driving portion 201 need to be detached, the second bolt and the third bolt are unscrewed, the first cover 20231 and the second cover 20233 can be detached, and then the driving portion 201 and the motor can be detached.

In the above embodiment, the hollow motor 4 can obtain electric power in the following two ways.

In the first mode, the driving handle 8 and the knife rod 101 are both provided with conducting ends; in the case where the driving handle 8 is connected to the knife rod 101, the conducting end of the driving handle 8 contacts with the conducting end inside the knife rod 101 to transmit the electric power of the driving handle 8 to the hollow motor 4 through the knife rod 101 to supply the hollow motor 4 with electric power.

In this way, the components for separately supplying power to the hollow motor 4 may be omitted, such as the motor power wire 7 and the like, thus making the structure of entire instrument more compact and lighter to operate.

In a second mode, as shown in FIG. 7, the ultrasonic knife further includes a motor power wire 7 connected to the hollow motor 4.

The structure of the whole instrument can be simplified by the implementation mode, and the instrument is convenient to manufacture.

The present invention has been illustrated by the above embodiments, but it should be understood that the above embodiments are for illustrative and descriptive purposes only and are not intended to limit the invention to the scope of the described embodiments. Furthermore, it will be understood by those skilled in the art that the present invention is not limited to the embodiments described above, and that many variations and modifications may be made in accordance with the teachings of the present invention, which variations and modifications are within the scope of the present invention as claimed. The protecting scope of the invention is defined by the appended claims and equivalents thereof.

## Claims

1. An ultrasonic knife, **characterized by** comprising a driving handle, a knife head assembly and a hollow motor;
wherein the knife head assembly comprises a knife rod and a knife head arranged at one end of the knife rod, a cavity is provided at the other end of the knife rod, an external thread is arranged at one end, close to the knife rod, of the driving handle, and an internal thread adapted with the external thread is arranged within the cavity;
a passage, through which the knife rod is able to pass, is provided inside the hollow motor, and the hollow motor is configured to drive the knife rod to rotate under a condition that the knife rod passes through the hollow motor.

2. The ultrasonic knife according to claim 1, wherein a camera assembly is further disposed on a portion of the knife rod, which is close to the knife head, the camera assembly comprises a camera portion, a driving portion and a transmission portion, the camera portion is connected with the driving portion through the transmission portion, and the driving portion is configured to drive the camera portion to rotate around the knife rod through the transmission portion.

3. The ultrasonic knife according to claim 2, wherein the transmission portion comprises a rotating sleeve arranged outside the knife rod, a moving gear and a stationary gear are provided inside the rotating sleeve, the stationary gear is sleeved outside the knife rod, the driving portion is arranged on the rotating sleeve, and the driving portion is connected with the moving gear; the camera portion is arranged on the peripheral wall of the rotating sleeve.

4. The ultrasonic knife according to claim 3, wherein the camera portion is detachably connected to the rotating sleeve.

5. The ultrasonic knife of claim 4, wherein the camera portion comprises a first bolt, a camera and a hoop sleeved outside the camera, the hoop is provided with a first screw hole adapted with the bolt, the rotating sleeve is provided with a second screw hole adapted with the bolt, and the first screw hole is connected with the second screw hole through the first bolt.

6. The ultrasonic knife according to claim 5, wherein the number of the second screw holes is at least two, and the at least two second screw holes are sequentially arranged from top to bottom along the axial direction of the rotating sleeve.

7. The ultrasonic knife according to claim 3, wherein the rotating sleeve comprises a first cover, a second cover and a peripheral wall, and the peripheral wall is detachably connected with the first cover and the second cover respectively.

8. The ultrasonic knife of claim 7, further comprising a second bolt and a third bolt; a third screw hole adapted with the third bolt is provided at one end face of the peripheral wall, a fourth screw hole adapted with the second bolt is provided at the other end face of the peripheral wall, a fifth screw hole adapted with the third bolt is provided at the first cover, and a sixth screw hole adapted with the third bolt is provided at the second cover; the third screw hole is connected with the fifth screw hole through the second bolt, and the fourth screw hole is connected with the sixth screw hole through the third bolt.

9. The ultrasonic knife according to claim 1, wherein a conducting end is provided on the driving handle and the knife rod;
under a condition that the driving handle is connected with the knife rod, the conducting end of the driving handle contacts with the conducting end in the knife rod, so as to transmit the electric energy of the driving handle to the hollow motor through the knife rod to supply power for the hollow motor.

10. The ultrasonic knife of claim 1, further comprising a motor power wire connected to the hollow motor.
